# EUROPÄISCHE PATENTANMELDUNG

(11) **EP 3 590 650 A1**
(43) Veröffentlichungstag der Anmeldung: **08.01.2020**
(21) Anmeldenummer: 19175718.6
(22) Anmeldetag: 21.05.2019
(51) Int. Cl.: B23K 26/70, E05D 3/02, E06B 7/36

(54) **SCHWENKBARES ECKELEMENT FÜR EINE STRAHLENSCHUTZWAND UND STRAHLENSCHUTZWAND**

(30) Priorität: 02.07.2018 DE 102018210869
(71) Anmelder: Laservision GmbH & Co. KG, 90766 Fürth (DE)
(72) Erfinder: Bescherer-Nachtmann, Klaus, 90419 Nürnberg (DE); Fröhlich, Thomas, 91207 Lauf an der Pegnitz (DE)
(74) Vertreter: Rau, Schneck & Hübner Patentanwälte Rechtsanwälte PartGmbB

(57) **Zusammenfassung**

Ein schwenkbares Eckelement (3) für eine Strahlenschutzwand weist mindestens ein eine Schwenkachse (9) definierendes Scharnier (8) und zwei über das mindestens eine Scharnier (8) verbundene Seitenteile (6, 7) auf, die relativ zueinander um die Schwenkachse (9) verschwenkbar sind. Beim Verschwenken der Seitenteile (6, 7) ergibt sich ein von einer jeweiligen Schwenkstellung abhängiger Zwischenraum (11) zwischen den Seitenteilen (6, 7). Das Eckelement (3) weist mindestens eine in dem Zwischenraum (11) angeordnete Blende (12, 13, 14) auf, wobei die mindestens eine Blende (12, 13, 14) derart gegenüber den Seitenteilen (6, 7) verlagerbar ist, dass die mindestens eine Blende (12, 13, 14) den Zwischenraum (11) unabhängig von der jeweiligen Schwenkstellung zumindest teilweise abdeckt.

## Beschreibung

Die vorliegende Patentanmeldung nimmt die Priorität der deutschen Patentanmeldung DE 10 2018 210 869.2 in Anspruch, deren Inhalt durch Bezugnahme hierin aufgenommen wird.

Die Erfindung betrifft ein schwenkbares Eckelement für eine Strahlenschutzwand, insbesondere eine Laserschutzwand. Die Erfindung betrifft weiterhin eine Strahlenschutzwand, insbesondere eine Laserschutzwand, mit einem derartigen schwenkbaren Eckelement.

Strahlenschutzwände werden zur Abschirmung von Strahlung, beispielsweise Röntgenstrahlung oder Laserstrahlung, verwendet. Strahlenschutzwände können schwenkbare Eckelemente aufweisen, um an den jeweiligen Einsatzort bzw. Einsatzzweck anpassbar zu sein.

Schwenkbare Eckelemente sind beispielsweise bekannt aus der DE 363 235 A und der GB 2 560 397 A.

Es ist die Aufgabe der vorliegenden Erfindung, ein verbessertes schwenkbares Eckelement für eine Strahlenschutzwand bereitzustellen, insbesondere ein Eckelement zu schaffen, das unabhängig von dessen Schwenkstellung undurchlässig für die Strahlung ist.

Diese Aufgabe wird erfindungsgemäß durch ein schwenkbares Eckelement mit den in Anspruch 1 angegebenen Merkmalen gelöst. Das schwenkbare Eckelement weist mindestens ein eine Schwenkachse definierendes Scharnier und zwei über das mindestens eine Scharnier verbundene Seitenteile auf. Die Seitenteile sind relativ zueinander um die Schwenkachse verschwenkbar, wobei sich ein von der jeweiligen Schwenkstellung der Seitenteile abhängiger Zwischenraum zwischen den Seitenteilen ergibt. Zudem ist mindestens eine in dem Zwischenraum zwischen den Seitenteilen angeordnete Blende vorhanden. Der Kern der Erfindung besteht darin, dass die mindestens eine Blende gegenüber den Seitenteilen verlagerbar ist, um den sich abhängig von der Schwenkstellung ergebenden Zwischenraum in jeder Schwenkstellung der Seitenteile zumindest teilweise abzudecken.

Aufgrund des sich beim Verschwenken der Seitenteile ergebenden und ändernden Zwischenraums können sich strahlendurchlässige Öffnungen, insbesondere Spalte im Bereich der Schwenkachse, zwischen den Seitenteilen bilden. Die verlagerbare mindestens eine Blende ist in ihrer Position bezüglich der Seitenteile an die jeweilige Schwenkstellung anpassbar. Die mindestens eine Blende deckt den sich ergebenden Zwischenraum zwischen den Seitenteilen zuverlässig ab. Bevorzugt deckt die mindestens eine Blende den Zwischenraum unabhängig von der jeweiligen Schwenkstellung der Seitenteile vollständig ab. Hierdurch werden strahlendurchlässige Öffnungen zuverlässig abgeschirmt. Ein Austreten von Strahlung ist vermindert, insbesondere vollständig vermieden, wodurch die Strahlensicherheit erhöht ist.

Die mindestens eine Blende ist ein Bestandteil des Eckelements. Die Abschirmung der Strahlung ist daher unabhängig von weiteren, gegebenenfalls nachträglich aufzubringenden Abdeckungen, wie beispielsweise Abdeckstoffen, gewährleistet. Die Installation der Strahlenschutzwand ist vereinfacht. Zudem ist ein unbeabsichtigtes Ablösen oder Verschieben von nachträglich aufgebrachten Abdeckungen, wodurch Strahlung entweichen kann, durch die in das Eckelement integrierte mindestens eine Blende vermieden. Die Strahlenschutzwirkung ist zuverlässig und sicher.

Die während des bestimmungsgemäßen Betriebs der Strahlenquelle zugewandten Oberflächen des Eckelements werden im Folgenden als Innenflächen bezeichnet. Diese Innenflächen können parallel zu weiteren der Strahlenquelle zugewandten Wandflächen der Strahlenschutzwand verlaufen. Das mindestens eine Scharnier ist bevorzugt auf der der Strahlenquelle zugewandten Seite, also auf der Seite der Innenflächen des Eckelements angeordnet.

Die Schwenkstellung der Seitenteile relativ zueinander kann durch einen Öffnungswinkel b quantifiziert werden. Der Öffnungswinkel b ist definiert als der Winkel, in welchem die beiden Innenflächen der Seitenteile zueinander stehen. Der Öffnungswinkel b = 180° definiert eine Geradestellung der Seitenteile. In der Geradestellung der Seitenteile liegen deren Innenflächen in einer gemeinsamen Ebene. Das Verschwenken der Seitenteile zueinander kann aus der Geradestellung in eine Schwenkstellung, für die b < 180° gilt, erfolgen. Abhängig von der Konstruktion des Scharniers und/oder der verlagerbaren Blende kann das Verschwenken bis zu einem minimalen Öffnungswinkel erfolgen. Der minimale Öffnungswinkel beträgt beispielsweise 135°, insbesondere 90°, insbesondere 45°, bevorzugt 0°. Ein kleiner Minimalwinkel ist insbesondere für ein platzsparendes Verstauen und Transportieren der Strahlenschutzwand vorteilhaft. Ein Öffnungswinkel b = 0° bedeutet, dass die Innenflächen der Seitenteile aufeinander zu liegen kommen. Die Seitenteile sind in diesem Fall vollständig umgeklappt.

In der Geradestellung kann der Zwischenraum zwischen den beiden Seitenteilen geschlossen sein. Der Zwischenraum kann sich für b < 180° öffnen. Insbesondere kann sich der Zwischenraum mit verkleinerndem Öffnungswinkel b vergrößern.

Die relativ zu den Seitenteilen verlagerbare mindestens eine Blende hat den Vorteil, dass die Abschirmung der Strahlung unabhängig von der Schwenkstellung der Seitenteile auch für kleine Öffnungswinkel b, insbesondere Öffnungswinkel b < 90°, gewährleistet ist. Die Verlagerung der mindestens einen Blende erfolgt bevorzugt abhängig von dem Öffnungswinkel b. Besonders bevorzugt ist die mindestens eine Blende um die Schwenkachse relativ zu den Seitenteilen verschwenkbar. Eine um die Schwenkachse verschwenkbare Blende kann optimal an die jeweilige Schwenkstellung der Seitenteile angepasst werden. Die Schutzwirkung der Strahlenschutzwand ist hierdurch weiter erhöht.

Gemäß einem vorteilhaften Aspekt der Erfindung ist die mindestens eine Blende mit dem mindestens einen Scharnier verbunden, insbesondere starr verbunden. Dies ermöglicht ein einfaches Verschwenken der mindestens einen Blende um die Schwenkachse. Insbesondere kann die mindestens eine Blende geführt um die Schwenkachse verschwenkt werden. Zudem ist eine direkte Verbindung der mindestens einen Blende mit den Seitenteilen nicht nötig. Dies gewährleistet ein leichtgängiges und zuverlässiges Verschwenken der mindestens einen Blende relativ zu den Seitenteilen.

Gemäß einem weiteren bevorzugten Aspekt der Erfindung sind mindestens zwei Blenden vorhanden, die relativ zueinander um die Schwenkachse verschwenkbar sind. Bevorzugt können die mindestens zwei Blenden fächerartig übereinander geschoben werden. Für große Öffnungswinkel b können die Blenden einander in Schwenkrichtung überlappen, insbesondere vollständig überlappen. Für kleine Öffnungswinkel b können die Blenden aufgefächert sein. Somit ist auch bei kleinen Öffnungswinkeln b der Zwischenraum zwischen den Seitenteilen zuverlässig abgeschirmt. Das Vorsehen mehrerer Blenden gewährleistet eine kompakte und besonders strahlendichte Ausführung des Eckelements. Bevorzugt sind drei Blenden vorgesehen.

Gemäß einem weiteren vorteilhaften Aspekt der Erfindung weist die mindestens eine Blende einen kreisbogenförmig um die Schwenkachse ausgeführten Blendenflügel auf. Ein kreisbogenförmig um die Schwenkachse ausgeführter Blendenflügel ist an die Schwenkbewegung um die Schwenkachse angepasst. Ein sich beim Verschwenken der Seitenteile ergebender Zwischenraum kann durch den kreisbogenförmigen Blendenflügel der mindestens einen Blende optimal abgedeckt sein. Neben dem kreisbogenförmig ausgebildeten Blendenflügel kann die mindestens eine Blende weitere Komponenten, beispielsweise Rückwände, aufweisen. Die mindestens eine Blende kann jedoch auch im Wesentlichen nur aus dem Blendenflügel bestehen. In diesem Fall kann die mindestens eine Blende insgesamt kreisbogenförmig um die Schwenkachse ausgeführt sein.

Bevorzugt sind mindestens zwei Blenden mit jeweils einem kreisbogenförmig um die Schwenkachse ausgeführten Blendenflügel vorgesehen. Die kreisbogenförmig ausgeführten Blendenflügel der mindestens zwei Blenden können leichtgängig und platzsparend beim Verschwenken übereinander geschoben werden.

Gemäß einem weiteren vorteilhaften Aspekt der Erfindung weisen die Seitenteile Blenden-Aufnahmen auf, die an die Kreisbogenform des Blendenflügels der mindestens einen Blende angepasst sind. Insbesondere bei großen Öffnungswinkeln, beispielsweise in der Geradestellung, kann die mindestens eine Blende, insbesondere deren Blendenflügel, zumindest zum Teil innerhalb der Blenden-Aufnahme gelagert sein. Durch die Blenden-Aufnahme ist eine kompakte Ausgestaltung des Eckelements möglich. Weiterhin ist zumindest der innerhalb der Blenden-Aufnahme gelagerte Teil der mindestens einen Blende vor Umwelteinflüssen geschützt. Vorteilhaft ist zudem, dass die mindestens eine Blende in den an die Kreisbogenform angepassten Aufnahmen geführt werden kann. Dies gewährleistet ein besonders stabiles und zuverlässiges Verschwenken.

Gemäß einem weiteren vorteilhaften Aspekt der Erfindung weist das Eckelement drei Blenden, nämlich zwei Seiten-Blenden und eine Mitten-Blende, auf. Die Seiten-Blenden weisen jeweils einen entlang eines gemeinsamen Seiten-Kreisbogens um die Schwenkachse ausgebildeten Seiten-Blendenflügel auf. Die Mitten-Blende weist einen entlang eines Mitten-Kreisbogens um die Schwenkachse ausgebildeten Mitten-Blendenflügel auf. Die Radien des Mitten-Kreisbogens und des Seiten-Kreisbogens unterscheiden sich. Die Seiten-Blendenflügel und der Mitten-Blendenflügel sind dadurch konzentrisch zueinander verschwenkbar und übereinanderschiebbar.

Die Seiten-Blenden können direkt benachbart zu je einem der Seitenteile angeordnet sein. Die Mitten-Blende kann zwischen den beiden Seiten-Blenden angeordnet sein. Bevorzugt ist der Radius des Seiten-Kreisbogens größer als der Radius des Mitten-Kreisbogens. Die Seiten-Blendenflügel können daher über den Mitten-Blendenflügel geschoben werden. Der Radius des Seiten-Kreisbogens ist besonders bevorzugt im Wesentlichen um die Dicke der Blendenflügel größer als der Radius des Mitten-Kreisbogens. Hierdurch weisen die Seiten-Blendenflügel einen kleinen radialen Abstand zu dem Mitten-Blendenflügel auf. Ein Austreten von Strahlung zwischen den Blendenflügeln ist zuverlässig vermieden.

Gemäß einem weiteren vorteilhaften Aspekt der Erfindung weist das Eckelement eine Mitnehmervorrichtung je Blende auf. Jede Mitnehmervorrichtung ist zur Mitnahme der jeweiligen Blende beim Verschwenken der Seitenteile ausgeführt, wenn der Öffnungswinkel b der Seitenteile kleiner als ein für die jeweilige Blende vorgegebener Mitnahmewinkel ist. Bei einer Mehrzahl von Blenden ist bevorzugt für jede Blende ein anderer Mitnahmewinkel vorgegeben. Der Mitnahmewinkel ist wie der Öffnungswinkel b definiert. Solange der Öffnungswinkel b größer als der Mitnahmewinkel der jeweiligen Blende ist, ist die Blende von der Schwenkbewegung der Seitenteile entkoppelt. Sobald der Öffnungswinkel b den jeweiligen Mitnahmewinkel unterschreitet, wird die Blende über die Mitnehmervorrichtung an die Schwenkbewegung der Seitenteile gekoppelt. Die Mitnahme der jeweiligen Blende erfolgt also dadurch, dass die Blende entsprechend der Schwenkbewegung der Seitenteile verlagert, insbesondere verschwenkt wird. Bevorzugt erfolgt die Mitnahme dadurch, dass die Blende synchron mit den Seitenteilen verschwenkt wird.

Die Mitnahme der mindestens einen Blende erfolgt bevorzugt nur bei Verkleinerung des Öffnungswinkels b. Die Mitnehmereinheit kann jedoch auch derart ausgeführt sein, dass eine Mitnahme der Blende auch beim Vergrößern des Öffnungswinkels b erfolgt, sofern der Öffnungswinkel b nicht größer als der jeweilige Mitnahmewinkel ist.

Gemäß einem weiteren bevorzugten Aspekt der Erfindung ist jede Mitnehmervorrichtung durch Anschläge in dem mindestens einen Scharnier ausgebildet. Eine derartige Ausführung der Mitnehmervorrichtung der mindestens einen Blende ist einfach, robust und platzsparend. Beispielsweise können je Scharnier zwei Blendenkupplungen vorgesehen sein. In den Blendenkupplungen können die Anschläge für jede Mitnehmervorrichtungen in unterschiedlichen Funktionsebenen angeordnet sein. Der Mitnahmewinkel kann durch einen Abstand der Anschläge in Schwenkrichtung auf einfache und präzise Weise definiert werden.

Gemäß einem weiteren bevorzugten Aspekt der Erfindung weisen die Seitenteile im Bereich des mindestens einen Scharniers jeweils einen Befestigungsblock auf. Die Befestigungsblöcke der Seitenteile können massiv ausgeführt sein. Zusätzlich können die Seitenteile Verkleidungsrahmen aufweisen. Die Verkleidungsrahmen können ein Hohlprofil ausbilden, in welchem die Befestigungsblöcke aufgenommen sind. Durch die Befestigungsblöcke ist eine stabile Ausführung des Eckelements, insbesondere im Bereich des mindestens einen Scharniers, gewährleistet. Gleichzeitig können die Seitenteile aufgrund der als Hohlprofil ausgebildeten Verkleidungsrahmen ein geringes Gewicht aufweisen.

Gemäß einem weiteren bevorzugten Aspekt der Erfindung weist der mindestens eine Befestigungsblock je Seitenteil in Richtung der Schwenkachse übereinander gestapelte Blechzuschnitte auf. Die übereinander gestapelten Blechzuschnitte ermöglichen eine hohe Flexibilität in der Ausgestaltung der Befestigungsblöcke. Die Befestigungsblöcke können beispielsweise durch das LOM-Verfahren (Laminated Object Modelling) gefertigt werden.

Das mindestens eine Scharnier kann durch die Blechzuschnitte der Befestigungsblöcke gebildet sein. Beispielsweise können die Blechzuschnitte der Befestigungsblöcke der beiden Seitenteile alternierend ineinandergreifen. Ein derartiges Scharnier ist stabil und ausfallsicher. Zudem können einzelne der Blechzuschnitte des Befestigungsblocks als Funktionsebenen der Blendenkupplung fungieren. Diese Blechzuschnitte können beispielsweise als Anschlagbleche für die Mitnehmervorrichtung ausgebildet sein.

Gemäß einem weiteren vorteilhaften Aspekt der Erfindung weisen die Seitenteile Befestigungselemente zum Befestigen weiterer Komponenten der Strahlenschutzwand, insbesondere zum Befestigen von Wandelementen auf. Bevorzugt sind die Befestigungselemente in den Befestigungsblöcken der Seitenteile verankert. Die Befestigungselemente ermöglichen insbesondere eine lösbare Verbindung der weiteren Komponenten der Strahlenschutzwand mit dem Eckelement. Dies ermöglicht einen modularen Aufbau der Strahlenschutzwand. Die Komponenten der Strahlenschutzwand können den jeweiligen Anforderungen entsprechend kombiniert werden.

Gemäß einem weiteren vorteilhaften Aspekt der Erfindung weist die mindestens eine Blende ein Strahlenschutzmaterial auf. Bevorzugt ist die mindestens eine Blende vollständig aus Strahlenschutzmaterial gefertigt. Besonders bevorzugt sind auch weitere Teile des Eckelements aus einem Strahlenschutzmaterial gefertigt. Das Strahlenschutzmaterial kann die jeweilige Strahlung insbesondere absorbieren. Hierdurch ist eine besonders hohe Schutzwirkung der Strahlenschutzwand gewährleistet.

Die Strahlenschutzwand kann insbesondere eine Laserschutzwand sein. In diesem Fall weisen die mindestens eine Blende und vorzugsweise auch weitere Komponenten des Eckelements ein Laserschutzmaterial auf. Geeignete Laserschutzmaterialien sind insbesondere Metalle, bevorzugt Aluminium oder Stahl. Alternativ können auch Graphit, Metall-Graphit-Verbundstoffe, Metall-Kunststoff-Verbundmaterialien und/oder geeignete Kunststoffe als Laserschutzmaterialien eingesetzt werden. Auch die Verwendung von Laserschutzglas ist möglich.

Es ist eine weitere Aufgabe der Erfindung, eine Strahlenschutzwand zu verbessern, insbesondere eine Strahlenschutzwand bereitzustellen, die flexibel einsatzbar und strahlenundurchlässig ist.

Diese Aufgabe wird gelöst durch eine Strahlenschutzwand mit den im Anspruch 13 genannten Merkmalen. Die Strahlenschutzwand weist mindestens zwei Wandelemente auf, die über ein schwenkbares Eckelement, wie es oben beschrieben ist, verbunden sind. Die beiden Wandelemente können über das schwenkbare Eckelement gegeneinander verschwenkt werden. Die Strahlenschutzwand ist flexibel und unabhängig von einer Schwenkstellung des Eckelements strahlenundurchlässig. Die weiteren Vorteile der Strahlenschutzwand ergeben sich aus den Vorteilen des schwenkbaren Eckelements.

Vorteilhafterweise ist die Strahlenschutzwand modular aufgebaut. Beispielsweise können weitere Wandelemente und/oder Eckelemente mit der Strahlenschutzwand verbunden werden. Die Strahlenschutzwand ist erweiterbar und kann einfach an den jeweiligen Anwendungsfall angepasst werden. Zudem können die Wandelemente lösbar mit dem Eckprofil verbunden sein, sodass die Wandelemente ausgetauscht werden können. Die Wandelemente selbst können aus in Rahmenprofilen eingesetzt Wandplatten bestehen. Beispielsweise können je nach abzuschirmender Strahlungsquelle Wandplatten aus unterschiedlichen Strahlenschutzmaterialien verwendet werden.

Nachfolgend werden unter Bezugnahme auf die beigefügten Zeichnungen bevorzugte Ausführungsformen der Erfindung beispielhaft beschrieben. Dabei zeigen:
- Fig. 1: eine perspektivische Ansicht eines ersten Ausführungsbeispiels einer Laserschutzwand mit zwei über ein Eckelement verbundenen Wandelementen,
- Fig. 2: ein Schnitt durch die Laserschutzwand gemäß Fig. 1 entlang der Schnittkante II-II in einer Geradestellung, wobei Wandplatten der Wandelemente der Übersichtlichkeit halber nicht dargestellt sind,
- Fig. 3: eine Seitenansicht von zwei Befestigungsblöcken und von Blenden des Eckelements der Laserschutzwand gemäß Fig. 1 in Geradestellung,
- Fig. 4: ein Detail IV der Seitenansicht gemäß Fig. 3,
- Fig. 5: eine Aufsicht auf die Befestigungsblöcke und Blenden gemäß Fig. 3 in einer Geradestellung, wobei Ankerplatten der Befestigungsblöcke der besseren Übersichtlichkeit halber nicht gezeigt sind,
- Fig. 6: eine Aufsicht auf die Befestigungsblöcke und Blenden gemäß Fig. 5 in einer Schwenkstellung, die einem Öffnungswinkel von 130° entspricht,
- Fig. 7: eine Aufsicht auf die Befestigungsblöcke und Blenden gemäß Fig. 5 in einer Schwenkstellung, die einem Öffnungswinkel von 90° entspricht, wobei ein Blockanschlagblech eines der Befestigungsblöcke der Übersichtlichkeit halber nicht gezeigt ist,
- Fig. 8: eine Aufsicht auf die Befestigungsblöcke und Blenden gemäß Fig. 7 in einer Schwenkstellung, die einem Öffnungswinkel von 50° entspricht,
- Fig. 9: eine Aufsicht auf die Befestigungsblöcke und Blenden gemäß Fig. 7 in einer Schwenkstellung, die einem Öffnungswinkel von 0° entspricht,
- Fig. 10: eine perspektivische Ansicht eines zweiten Ausführungsbeispiels einer Laserschutzwand in einer Schwenkstellung, die einem Öffnungswinkel von 150° entspricht, und
- Fig. 11: eine perspektivische Ansicht der Laserschutzwand gemäß Fig. 10 in einer Schwenkstellung, die einem Öffnungswinkel von 30° entspricht.

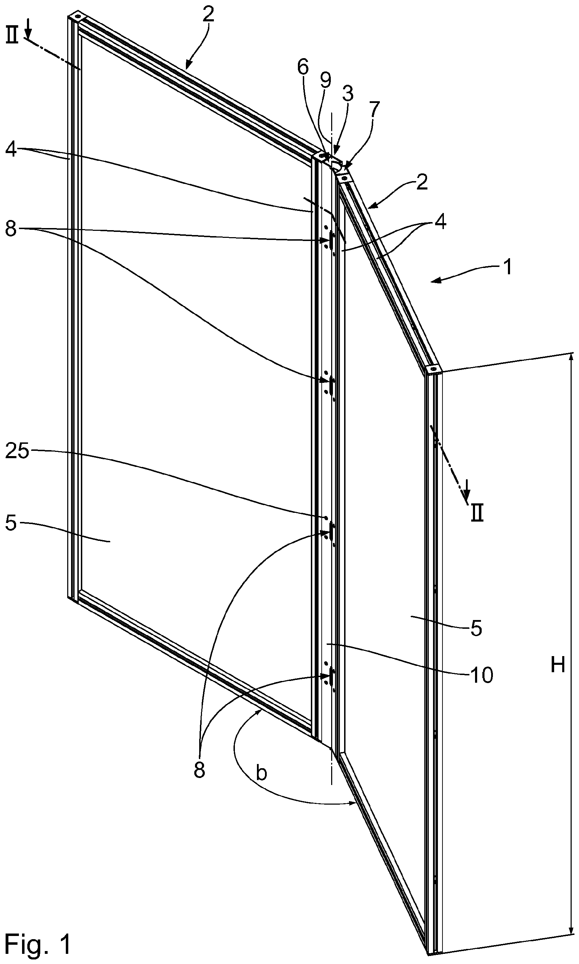

Mit Bezugnahme auf die Fig. 1 bis 9 wird ein erstes Ausführungsbeispiel einer Strahlenschutzwand 1 beschrieben. Die Strahlenschutzwand 1 ist eine Laserschutzwand. Die Laserschutzwand 1 weist zwei Wandelemente 2 und ein die Wandelemente 2 verbindendes Eckelement 3 auf. Die Wandelemente 2 weisen Rahmenprofile 4 und darin befestigte Wandplatten 5 auf.

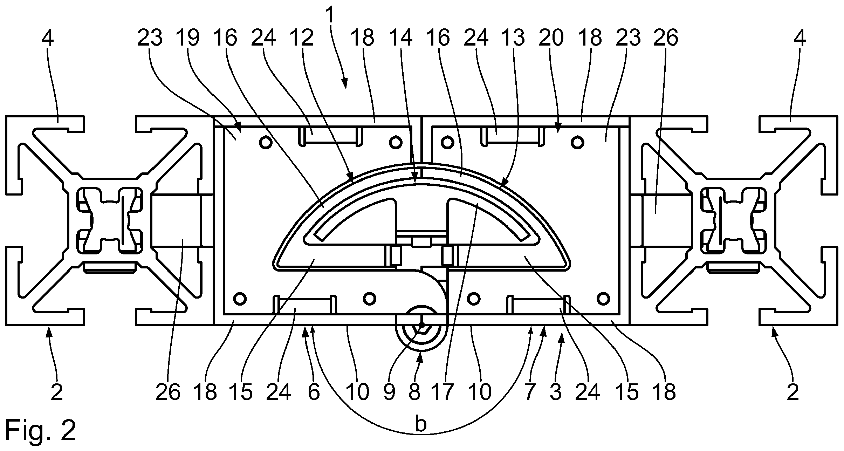

Die Rahmenprofile 4 sind umfangseitig um die Wandplatten 5 angeordnet und dienen zur Verbindung der Wandelemente 2 mit dem Eckelement 3 bzw. mit weiteren Wandelementen. Über die Rahmenprofile 4 ist die Laserschutzwand 1 mit weiteren Elementen erweiter- und kombinierbar. Die Laserschutzwand 1 weist einen modularen Aufbau auf. Der Übersichtlichkeit halber sind in Fig. 2 nur die dem Eckelement 3 benachbarten Rahmenelemente 4 und nicht die weiteren Teile der Wandelemente 2, insbesondere nicht die Wandplatten 5, gezeigt.

Das Eckelement 3 umfasst ein erstes Seitenteil 6 und ein zweites Seitenteil 7. Das erste Seitenteil 6 ist mit dem zweiten Seitenteil 7 über mehrere Scharniere 8 verbunden. Die Scharniere 8 definieren eine gemeinsame Schwenkachse 9. Die Laserschutzwand 1 weist in Richtung der Schwenkachse 9 einen Höhe H auf. Die Scharniere 8 sind entlang der Schwenkachse 9 gleichmäßig über die Höhe H verteilt. Die Seitenteile 6, 7 und die jeweils hiermit verbundenen Wandteile 2 sind um die Schwenkachse 9 relativ zueinander verschwenkbar. Das Eckelement 3 wird auch als schwenkbares Eckelement 3 bezeichnet.

Die Laserschutzwand 1 dient zum Abschirmen von Laserstrahlung einer nicht dargestellten Laserstrahlungsquelle. Die Laserstrahlungsquelle ist auf der Seite der Laserschutzwand 1 angeordnet, auf der sich die Scharniere 8 befinden. Die der Laserstrahlungsquelle zugewandten Oberflächen des Eckelements 3 werden als Innenflächen 10 (vgl. Fig. 2) der Seitenteile 6, 7 bezeichnet. Die Innenflächen 10 der Seitenteile 6, 7 sind parallel zu den der Laserstrahlungsquelle zugewandten Oberflächen der Wandplatten 5.

Eine jeweilige Schwenkstellung der Seitenteile 6, 7 zueinander wird durch einen Öffnungswinkel b quantifiziert. Der Öffnungswinkel b ist definiert als der Winkel, in welchem die Innenflächen 10 der Seitenteile 6, 7 zueinander stehen. In Fig. 2 sind die Seitenteile 6, 7 des Eckelements 3 und damit auch die Wandelemente 2 in einer Geradestellung dargestellt. In der Geradestellung beträgt der Öffnungswinkel b = 180°. In der Geradestellung liegen die Innenflächen 10 der Seitenteile 6, 7 in einer Ebene. Für die von der Geradestellung verschiedenen Schwenkstellungen gilt b < 180°. In Fig. 1 ist beispielsweise eine Schwenkstellung dargestellt, deren entsprechender Öffnungswinkel b = 130° beträgt.

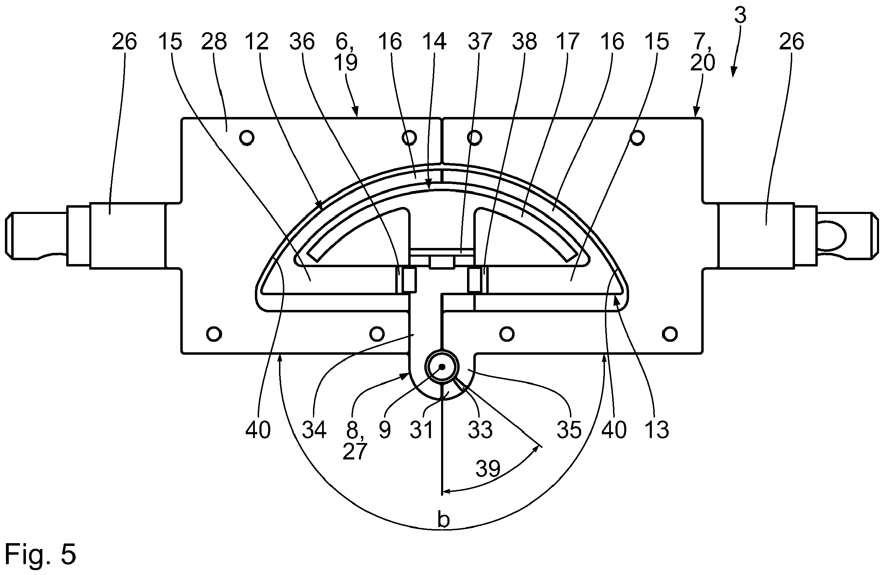

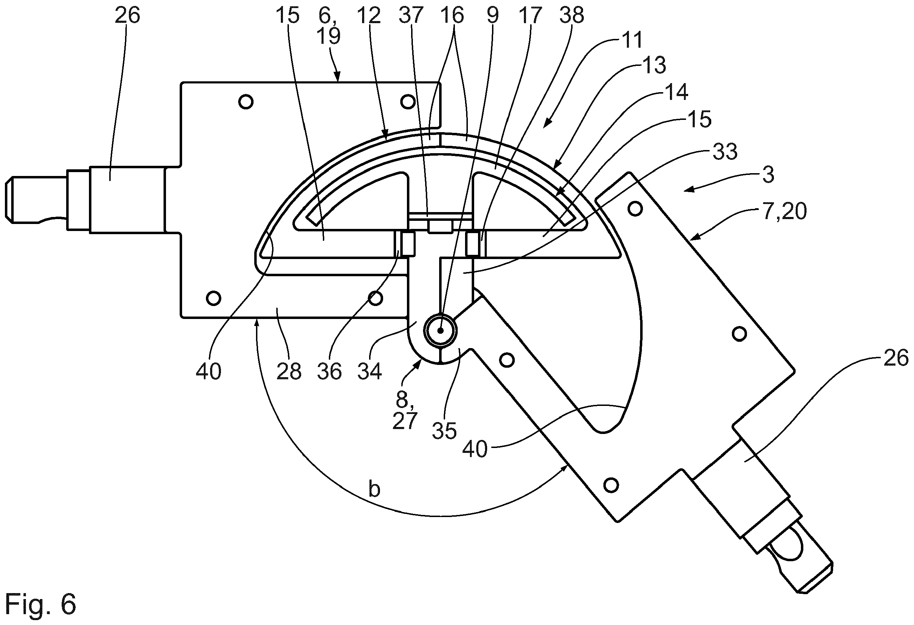

Beim Verschwenken der Seitenteile 6, 7 zueinander ergibt sich ein von der jeweiligen Schwenkstellung abhängiger Zwischenraum 11 zwischen den Seitenteilen 6, 7. In der Geradestellung ist der Zwischenraum 11 geschlossen. In den die Geradestellung zeigenden Figuren (vgl. insbesondere Fig. 2 und 5) ist der Zwischenraum 11 entsprechend nicht angezeichnet. Für b < 180 öffnet sich der Zwischenraum 11 zwischen den Seitenteilen 6, 7 (siehe Fig. 6 bis 9). Um zu verhindern, dass Laserstrahlung durch den Zwischenraum 11 austreten kann, sind in dem Zwischenraum 11 eine erste Seiten-Blende 12, eine zweite Seiten-Blende 13 und eine Mitten-Blende 14 angeordnet. Die Blenden 12, 13, 14 erstrecken sich über die gesamte Höhe H der Laserschutzwand. Sie sind derart relativ zueinander und relativ zu den Seitenteilen 6, 7 um die Schwenkachse 9 verschwenkbar, dass der Zwischenraum 11 unabhängig von einer Schwenkstellung der Seitenteile 6, 7 durch die Blenden 12, 13, 14 vollständig abgedeckt wird. Die Blenden 12, 13, 14 schirmen die Laserstrahlung unabhängig von der Schwenkstellung der Seitenteile 6, 7 zuverlässig ab.

Die Seiten-Blenden 12, 13 weisen jeweils Rückwände 15 und hiermit verbundene Seiten-Blendenflügel 16 auf. Die Seiten-Blendenflügel 16 sind kreisbogenförmig entlang eines gemeinsamen Seiten-Kreisbogens um die Schwenkachse 9 ausgebildet. Auch die Mitten-Blende 14 weist einen Mitten-Blendenflügel 17 auf, der kreisbogenförmig entlang eines Mitten-Kreisbogens um die Schwenkachse 9 verläuft. Die durch die Seiten-Blendenflügel 16 und den Mitten-Blendenflügel 17 beschriebenen Kreisbögen sind konzentrisch. Der Mitten-Kreisbogen weist einen im Vergleich zu dem Seiten-Kreisbogen verringerten Radius auf. Der Unterschied der Radien entspricht im Wesentlichen einer Dicke der Blendenflügel 16, 17 in radialer Richtung. Durch die kreisbogenförmige Ausgestaltung der Blendenflügel 16, 17 und die unterschiedlichen Radien können die Blendenflügel 16, 17 der Blenden 12, 13, 14 übereinander geschoben werden. Insbesondere kann die Mitten-Blende 14 in einem aus den Seiten-Blendenflügel 16 und den Rückwänden 15 gebildeten Freiraum aufgenommen werden.

Im Folgenden wird der Aufbau der Seitenteile 6, 7 und deren Zusammenwirken mit den Blenden 12, 13, 14 beim Verschwenken beschrieben. Die Seitenteile 6, 7 weisen jeweils sich über die gesamte Höhe H der Laserschutzwand 1 erstreckende Verkleidungsrahmen 18 auf. Im Bereich der Scharniere 8 weist das erste Seitenteil 6 jeweils einen ersten Befestigungsblock 19 und das zweite Seitenteil 7 jeweils einen zweiten Befestigungsblock 20 auf.

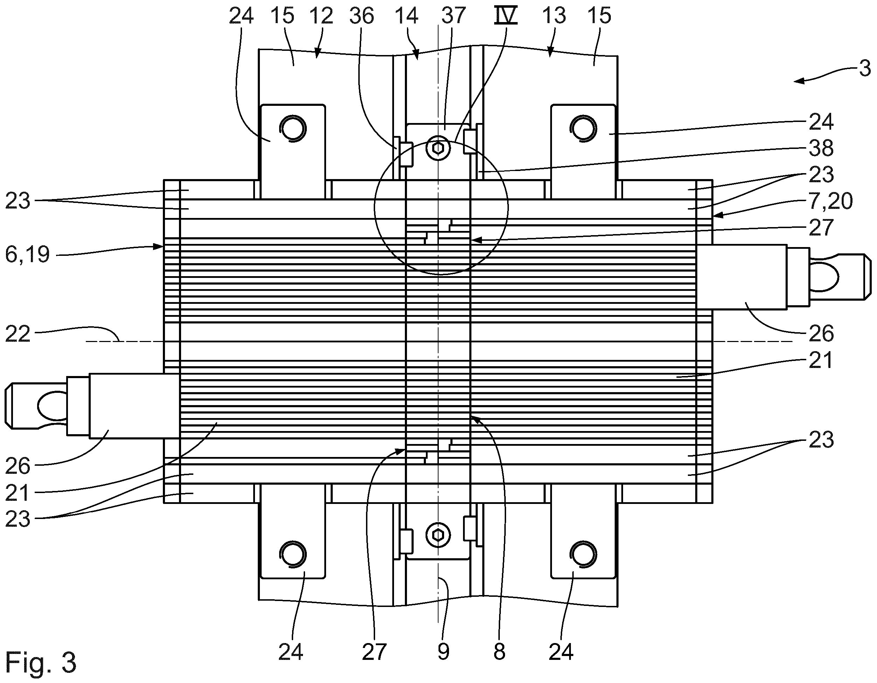

Fig. 3 zeigt eine Seitenansicht eines der Befestigungsblöcke 19 des ersten Seitenteils 6 und eines der Befestigungsblöcke 20 des zweiten Seitenteils 7 sowie der Blenden 12, 13, 14 in der Geradestellung. Die Befestigungsblöcke 19, 20 weisen einen Schichtaufbau aus mehreren in Richtung der Schwenkachse 9 übereinander gestapelten Blechzuschnitten 21 auf. Ein derartiger Schichtaufbau kann beispielsweise durch das sogenannte LOM-Verfahren (Laminated Object Modelling) gefertigt werden. Der Schichtaufbau des zweiten Befestigungsblocks 20 ist bezüglich einer senkrecht zu der Schwenkachse 9 verlaufenden Mittelebene 22 spiegelsymmetrisch zu dem Schichtaufbau des ersten Befestigungsblocks 19 ausgeführt. Die Scharniere 8 sind aus den Blechzuschnitten 21 der jeweiligen Befestigungsblöcke 19, 20 gebildet. Hierzu überlappen sich im Bereich des jeweiligen Scharniers 8 zumindest bereichsweise die Blechzuschnitte 21 des ersten Befestigungsblocks 19 alternierend mit den Blechzuschnitten 21 des zweiten Befestigungsblocks 20. Die Befestigungsblöcke 19, 20 weisen jeweils in Richtung der Schwenkachse 9 randseitig gelegene Ankerplatten 23 mit Ankervorsprüngen 24 auf. Die Ankervorsprünge 24 weisen Schraubbohrungen auf, worüber die Befestigungsblöcke 19, 20 mit den Verkleidungsrahmen 18 mithilfe von Ankerschrauben 25 (vgl. Fig. 1) verschraubt werden können.

Jeder Befestigungsblock 19, 20 weist je ein Befestigungselement 26 auf. Die Befestigungselemente 26 stehen in Richtung der jeweiligen Wandelemente 2 über die Befestigungsblöcke 19, 20 und die Verkleidungsrahmen 18 über. Die Befestigungselemente 26 dienen zur Befestigung der Rahmenprofile 4 der Wandelemente 2 an den Eckelementen 3.

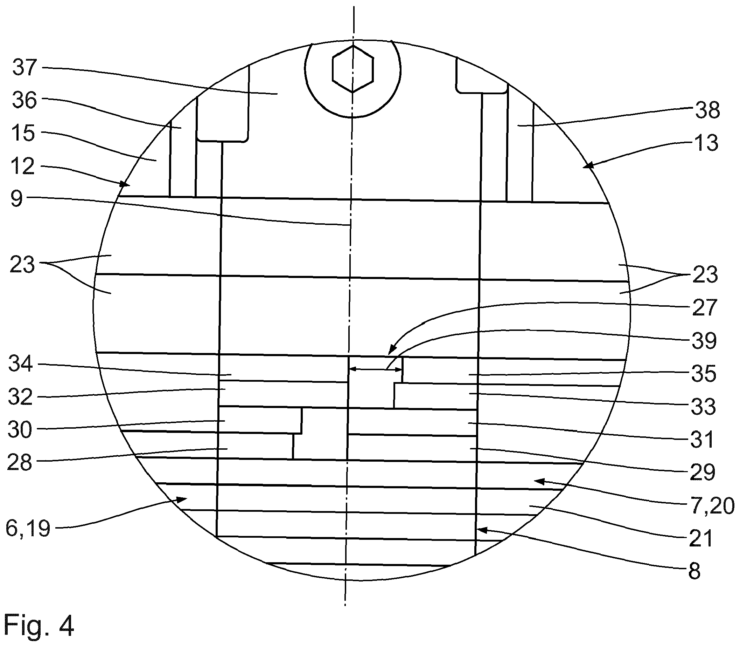

Die Blenden 12, 13, 14 sind mit den Scharnieren 8 jeweils über Blendenkupplungen 27 verbunden. Je Scharnier 8 sind der Symmetrie der Befestigungsblöcke 19, 20 entsprechend zwei Blendenkupplungen 27 vorgesehen. Die Blendenkupplungen 27 sind Teil der Scharniere 8. Die Blendenkupplungen 27 sind zwischen den Ankerplatten 23 und weiteren Blechzuschnitten 21 der Befestigungsblöcke 19, 20 angeordnet. In Fig. 4 ist eine Seitenansicht einer Blendenkupplung 27 im Detail gezeigt. Die Blendenkupplung 27 weist vier in Richtung der Schwenkachse 9 übereinanderliegende Funktionsebenen auf. In jeder Funktionsebene liegen sich bezogen auf die Schwenkrichtung zwei Anschlagbleche gegenüber. In einer ersten Funktionsebene ist ein erstes Block-Anschlagblech 28 vorhanden. Dem ersten Block-Anschlagblech 28 gegenüber liegend ist ein erstes Seiten-Anschlagblech 29 angeordnet. In der zweiten Funktionsebene liegen sich ein zweites Seiten-Anschlagblech 30 und ein erstes Mitten-Anschlagblech 31 gegenüber. In der dritten Funktionsebene liegen sich ein zweites Mitten-Anschlagblech 32 und ein drittes Seiten-Anschlagblech 33 gegenüber. In der vierten Funktionsebene liegen sich ein viertes Seiten-Anschlagblech 34 und ein zweites Block-Anschlagblech 35 gegenüber. Das erste Block-Anschlagblech 28 ist ein Blechzuschnitt des ersten Befestigungsblocks 19. Das zweite Block-Anschlagblech 35 ist ein Blechzuschnitt des zweiten Befestigungsblocks 20. Das erste Seiten-Anschlagblech 29 und das zweite Seiten-Anschlagblech 30 sind über einen ersten Seiten-Anker 36 mittels eine Schraubverbindung starr mit der Rückwand 15 der ersten Seiten-Blende 12 verbunden. Die Mitten-Anschlagbleche 31, 32 sind über einen Mitten-Anker 37 starr mit der Mitten-Blende 14 verbunden. Ebenso sind das dritte Seiten-Anschlagblech 33 und das vierte Seiten-Anschlagblech 34 über einen zweiten Seiten-Anker 38 starr mit der Rückwand 15 der zweiten Seiten-Blende 13 verschraubt. Die einander gegenüberliegenden Anschlagbleche bilden Anschläge, die eine Kopplung der Schwenkbewegungen der starr mit den jeweiligen Anschlagblechen verbundenen Komponenten bewirkt. Beispielsweise wird in der ersten Funktionsebene durch das Anschlagen des ersten Block-Anschlagblechs 28 an das erste Seiten-Anschlagblech 29 der erste Befestigungsblock 19 mit der ersten Seiten-Blende 12 gekoppelt. Durch die in den verschiedenen Funktionsebenen ausgebildeten Anschläge ist daher jeweils eine Mitnehmervorrichtung geschaffen, die die jeweiligen Blenden 12, 13, 14 beim Verschwenken der Seitenteile 6, 7 zueinander mitnehmen kann.

Zwischen den sich in den einzelnen Funktionsebenen gegenüberliegenden Anschlagblechen ist in der in Fig. 4 gezeigten Geradestellung jeweils ein Schwenkabstand vorhanden. In Fig. 4 ist ein beispielhafter Schwenkabstand 39 zwischen dem vierten Seiten-Anschlagblech 34 und dem zweiten Block-Anschlagblech 35 gezeigt. Die Schwenkabstände definieren einen Freilauf, der ein Verschwenken des einen Anschlagblechs relativ zu dem in der jeweiligen Funktionsebene gegenüberliegenden Anschlagblech ermöglicht, bevor diese aneinanderschlagen und eine Kopplung der Schwenkbewegung bewirken. Die Schwenkabstände definieren daher einen für die jeweilige Blenden 12, 13, 14 unterschiedlichen Mitnahmewinkel, ab welchem die Bewegung der einander gegenüber liegenden Anschlagbleche gekoppelt ist.

Die Funktionweise der Blendenkupplung 27 und der verschwenkbaren Blenden 12, 13, 14 wird im Folgenden anhand der Fig. 5 bis 9 erläutert. Diese Figuren zeigen jeweils eine Aufsicht auf die Befestigungsblöcke 19, 20 und die Blenden 12, 13, 14 in verschiedenen Schwenkstellungen. In den Figuren sind die Ankerplatten 23 mit den Ankervorsprüngen 24 nicht dargestellt, sodass Details der Blendenkupplung 27 zu erkennen sind.

In Fig. 5 sind die Befestigungsblöcke 19, 20 und die Blenden 12, 13, 14 in Geradestellung gezeigt. Der Öffnungswinkel b beträgt 180°. Die Aufsicht zeigt das erste Block-Anschlagblech 28 des ersten Befestigungsblocks 19 und das zweite Block-Anschlagblech 35 des zweiten Befestigungsblocks 20. In der Blendenkupplung 27 liegt dem zweiten Block-Anschlagblech 35 das vierte Seiten-Anschlagblech 34 gegenüber, das über den zweiten Seiten-Anker 38 starr mit der zweiten Seiten-Blende 13 verbunden ist. Zwischen dem vierten Seiten-Anschlagblech 34 und dem zweiten Block-Anschlagblech 35 ist der Schwenkabstand 39 angezeichnet. Im Bereich des Schwenkabschnitts 39 ist ein Teil des dritten Seiten-Anschlagblechs 33 und des ersten Mitten-Anschlagblech 31 der darunterliegenden Funktionsebenen erkennbar.

In der in der Fig. 5 gezeigten Geradestellung sind die Blendenflügel 16, 17 maximal übereinander geschoben. Insbesondere kommt die Mitten-Blende 14 in einem Freiraum zwischen den Seiten-Blendenflügeln 16 und den Rückwänden 15 der Seiten-Blenden 12, 13 zu liegen. In der Geradestellung ist der Zwischenraum 11 zwischen den Seitenteilen 6, 7 geschlossen. Die Blenden 12, 13, 14 sind vollständig innerhalb von Blenden-Aufnahmen 40 der Seitenteile 6, 7 gelagert. Die Blenden-Aufnahmen 40 weisen eine an die Kreisbogenform der Seiten-Blendenflügel 16 angepassten Kontur auf. Sie sind innerhalb der Befestigungsblöcke 19, 20 ausgebildet.

In Fig. 6 ist eine Aufsicht auf die Befestigungsblöcke 19, 20 und die Blenden 12, 13, 14 gezeigt. Der zweite Befestigungsblock 20 ist gegenüber dem ersten Befestigungsblock 19 und den Blenden 12, 13, 14 verschwenkt. Die gezeigte Schwenkstellung entspricht einem Öffnungswinkel b = 130°. Zwischen den Seitenteilen 6, 7 bzw. zwischen deren Befestigungsblöcken 19, 20 hat sich der Zwischenraum 11 geöffnet. In der dargestellten Schwenkstellung schlägt das zweite Block-Anschlagblech 35 des zweiten Befestigungsblocks 20 an das vierte Seiten-Anschlagblech 34 an. Hierdurch wird die zweite Seiten-Blende 13, die starr mit dem vierten Seiten-Anschlagblech 34 verbunden ist, mit dem zweiten Befestigungsblock 20 gekoppelt. Der Öffnungswinkel b = 130° entspricht einem Mitnahmewinkel für die zweite Seiten-Blende 13. Wird der Öffnungswinkel b weiter verringert, wird gleichermaßen auch die zweite Seiten-Blende 13 verschwenkt. Bei einer Vergrößerung des Öffnungswinkels b hingegen wird die Kopplung gelöst.

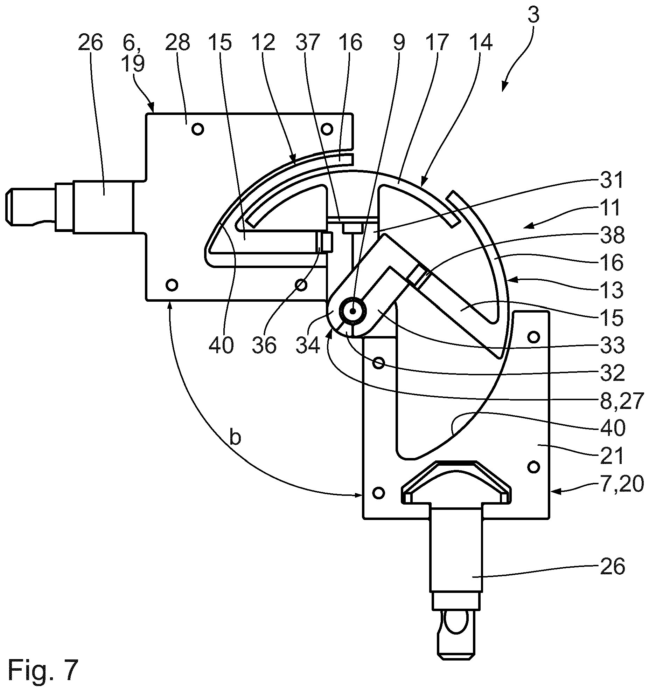

Fig. 7 zeigt eine Aufsicht auf die Befestigungsblöcke 19, 20 und die Blenden 12, 13, 14 in einer Schwenkstellung, die einem Öffnungswinkel b von 90° entspricht. In der gezeigten Schwenkstellung ist der zweite Befestigungsblock 20 noch weiter gegenüber dem ersten Befestigungsblock 19 verschwenkt. Der Zwischenraum 11 ist weiter vergrößert. Da b = 90° kleiner als der Mitnahmewinkel der zweiten Seiten-Blende 13 ist, ist auch die zweiten Seiten-Blende 13 gegenüber dem ersten Befestigungsblock 19 und den weiteren Blenden 12, 14 verschwenkt. Aufgrund der verschwenkten zweiten Seiten-Blende 13 ist der Zwischenraum 11 weiterhin vollständig von den Blenden 12, 13, 14 abgedeckt.

In den Fig. 7 bis 9 ist das zweite Block-Anschlagblech 35 des zweiten Befestigungsblocks 20 nicht dargestellt. Hierdurch sind weitere Details der Blendenkupplung 27 zu erkennen. Für den Öffnungswinkel b = 90° schlägt das dritte Seiten-Anschlagblech 33 an das zweite Mitten-Anschlagblech 32 an. Der Öffnungswinkel b = 90° entspricht daher einem Mitnahmewinkel für die Mittenblende, die starr mit dem zweiten Mitten-Anschlagblech 32 verbunden ist. Wird der zweite Befestigungsblock 20 weiter gegenüber dem ersten Befestigungsblock 19 verschwenkt, d.h. wird der Öffnungswinkel b weiter verringert, wird auch die Mitten-Blende 14 zusammen mit der zweiten Seiten-Blende 13 und dem zweiten Befestigungsblock 20 gegenüber dem ersten Befestigungsblock 19 und der ersten Seiten-Blende 12 verschwenkt.

Aufgrund des in Fig. 7 nicht gezeigten zweiten Block-Anschlagblechs 35 ist ein tieferliegender Blechzuschnitt 21 des zweiten Befestigungsblocks 20 zu sehen, in dem das Befestigungselement 26 verankert ist.

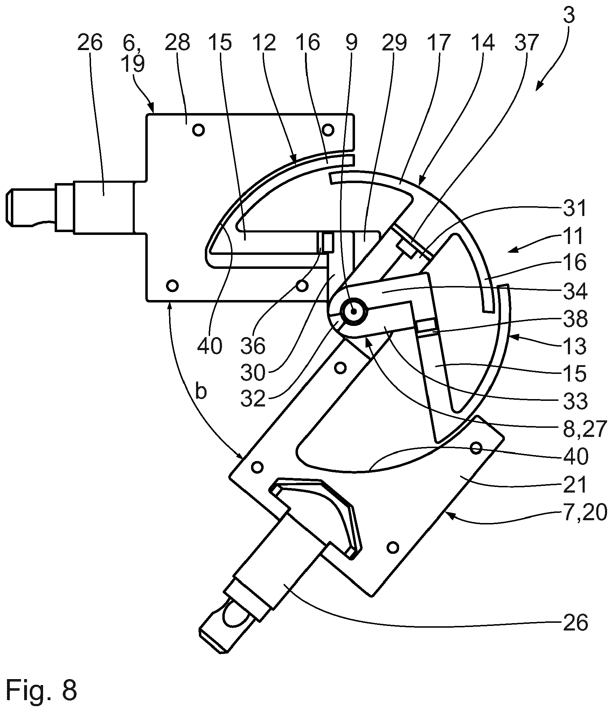

In Fig. 8 ist eine Aufsicht auf die Befestigungsblöcke 19, 20 und die Blenden 12, 13, 14 in einer Schwenkstellung gezeigt, die einem Öffnungswinkel b = 50° entspricht. In der gezeigten Schwenkstellung ist der Befestigungsblock 20 noch weiter gegenüber dem Befestigungsblock 19 verschwenkt, der Zwischenraum 11 ist weiter vergrößert. Da der Öffnungswinkel b = 50° kleiner als die Mitnahmewinkel für die zweite Seiten-Blende 13 und die Mitten-Blende 14 ist, sind auch die zweite Seiten-Blende 13 und die Mitten-Blende 14 gegenüber dem ersten Befestigungsblock 19 und der ersten Seiten-Blende 12 verschwenkt. Hierdurch ist der Zwischenraum 11 weiterhin vollständig durch die Blenden 12, 13, 14 abgedeckt.

Die verschwenkte Mitten-Blende 14 gibt den Blick auf das erste Seiten-Anschlagblech 29 und das zweite Seiten-Anschlagblech 30 frei, die über den ersten Seiten-Anker 36 starr mit der ersten Seiten-Blende 12 verbunden sind. Der Öffnungswinkel b = 50° entspricht dem Mitnahmewinkel für die erste Seiten-Blende 12. Wird der zweite Befestigungsblock 20 weiter gegenüber dem ersten Befestigungsblock 19 verschwenkt, d.h. wird der Öffnungswinkel b weiter verringert, wird auch die erste Seiten-Blende 12 zusammen mit den weiteren Blenden 13, 14 und dem zweiten Befestigungsblock 20 gegenüber dem ersten Befestigungsblock 19 verschwenkt.

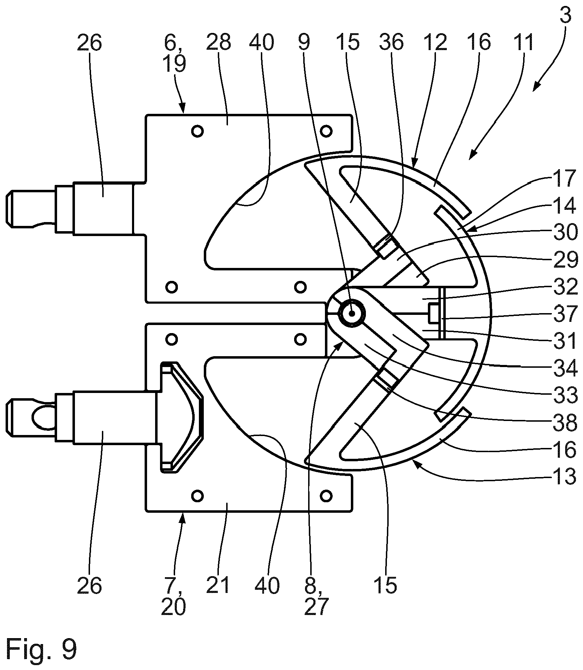

In Fig. 9 ist eine Aufsicht auf die Befestigungsblöcke 19, 20 und die Blenden 12, 13, 14 gezeigt. Die Befestigungsblöcke 19, 20 sind maximal zueinander verschwenkt, sodass für den Öffnungswinkel b = 0° gilt. Wären die Verkleidungsrahmen 18 der Seitenteile 6, 7 dargestellt, würden deren Innenflächen 10 aufeinander zu liegen kommen. In der gezeigten Schwenkstellung sind alle drei Blenden 12, 13, 14 relativ zueinander und relativ zu den Befestigungsblöcken 19, 20 verschwenkt. Der Zwischenraum 11 ist weiterhin zuverlässig abgedeckt.

In den verschiedenen in den Fig. 5 bis 9 gezeigten Schwenkstellungen ergibt sich jeweils ein unterschiedlich großer Zwischenraum 11 zwischen den Seitenteilen 6, 7 bzw. deren Befestigungsblöcken 19, 20. Mithilfe der Blendenkupplungen 27 werden die Blenden abhängig von dem jeweiligen Öffnungswinkel b gezielt verschwenkt, sodass diese den Zwischenraum 11 jeweils vollständig abdecken. Laserstrahlung kann unabhängig von der Schwenkstellung und dem Öffnungswinkel b nicht durch das Eckelement 3 entweichen.

Die Wandelemente 2 und das Eckelement 3, insbesondere die Wandplatten 5 und die Blendenflügel 16, 17 sind aus Laserschutzmaterial gefertigt. Beispielhafte Laserschutzmaterialien sind Metalle, insbesondere Aluminium oder Stahl, Graphit, Metall-Graphit-Verbundwerkstoffe, Metall-Kunststoff-Verbundmaterialien und/oder Glas, bevorzugt Laserschutzglas. Auch die Verwendung von Kunststoffen mit geeigneter mechanischer Festigkeit und entsprechender Strahlenschutzwirkung ist möglich.

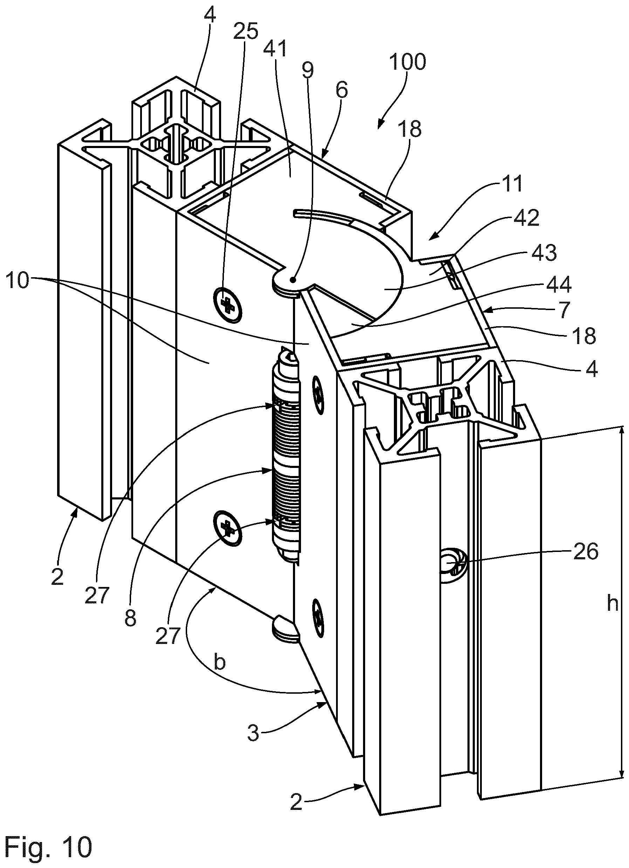

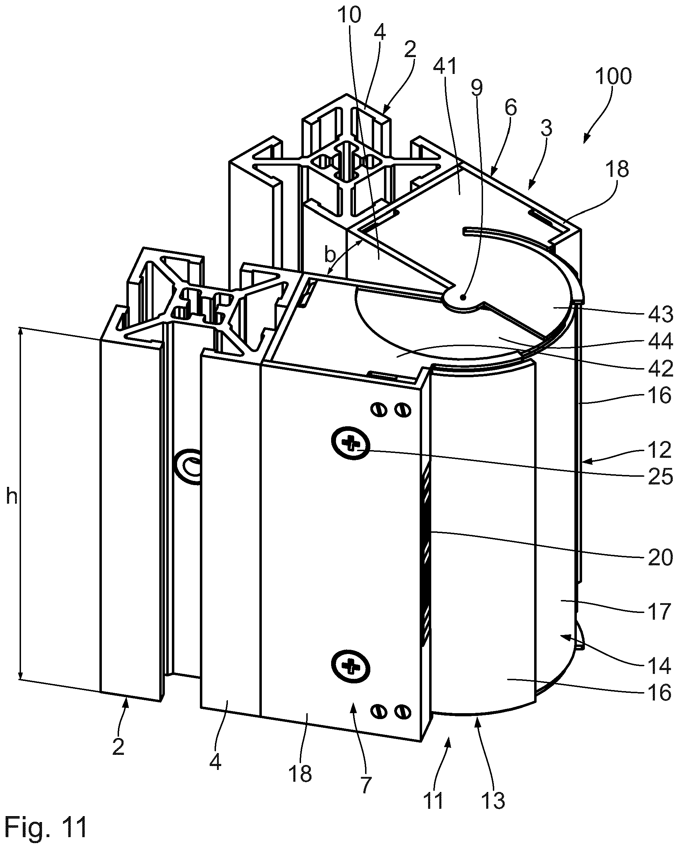

In den Fig. 10 und 11 ist ein zweites Ausführungsbeispiel einer Laserschutzwand 100 gezeigt. Komponenten, die vorstehend unter Bezugnahme auf die Fig. 1 bis 9 bereits beschrieben wurden, tragen die gleichen Bezugszeichen und werden nicht noch einmal im Einzelnen diskutiert.

Die Laserschutzwand 100 unterscheidet sich von der Laserschutzwand 1 lediglich in ihrer geringen Höhe h in Richtung der Schwenkachse 9. Aufgrund der geringen Höhe h weist das Eckelement 3 der Laserschutzwand 100 lediglich ein Scharnier 8 auf.

Aus Gründen der Übersichtlichkeit sind in den Fig. 10 und 11 die Wandplatten 5 nicht dargestellt. Die Fig. 10 zeigt eine perspektivische Darstellung der Laserschutzwand 100 für eine Schwenkstellung, die einem Öffnungswinkel b = 150° entspricht. In Fig. 11 ist die Laserschutzwand 100 für einen Öffnungswinkel b = 30° gezeigt.

Das Eckelement 3 der Laserschutzwand 100 weist an den Stirnseiten angebrachte Stirnverkleidungselemente 41, 42 auf. Das Eckelement 3 der Laserschutzwand 1 weist entsprechende Stirnverkleidungselemente auf, die in Fig. 1 aus Gründen der Übersichtlichkeit jedoch nicht mit Bezugszeichen versehen sind. Das Stirnverkleidungselement 41 ist dem ersten Seitenteil 6 zugeordnet. Das zweite Stirnverkleidungselement 42 ist dem zweiten Seitenteil 7 zugeordnet. Die Stirnverkleidungselemente 41, 42 weisen übereinander schiebbare kreissektorförmige Deckplatten 43, 44 auf. Hierdurch ist eine stirnseitige Verkleidung des Eckelements 3 unabhängig von der Schwenkstellung gewährleistet. Auch stirnseitig kann keine Laserstrahlung entweichen.

In weiteren nicht dargestellten Ausführungsbeispielen sind weniger als drei oder mehr als drei zueinander verschwenkbare Blenden vorgesehen. Beispielsweise kann eine einzige Blende, deren Blendenflügel einen großen Kreisbogen abdeckt, genügen, um ein Verschwenken der Seitenteile über einen großen Öffnungswinkel hinweg zu ermöglichen. Für den Fall, dass N Blenden vorgesehen sind, weisen die Blendenkupplungen der Scharniere N+1 Funktionsebenen auf.

## Patentansprüche

1. Schwenkbares Eckelement für eine Strahlenschutzwand, insbesondere Laserschutzwand, aufweisend
- mindestens ein eine Schwenkachse (9) definierendes Scharnier (8),
- zwei über das mindestens eine Scharnier (8) verbundene Seitenteile (6, 7), die relativ zueinander um die Schwenkachse (9) verschwenkbar sind, wobei sich ein von einer jeweiligen Schwenkstellung der Seitenteile (6, 7) abhängiger Zwischenraum (11) zwischen den Seitenteilen (6, 7) ergibt, und
- mindestens eine in dem Zwischenraum (11) zwischen den Seitenteilen (6, 7) angeordnete Blende (12, 13, 14),
wobei die mindestens eine Blende (12, 13, 14) gegenüber den Seitenteilen (6, 7) verlagerbar ist, um den sich abhängig von der Schwenkstellung der Seitenteile (6, 7) ergebenden Zwischenraum (11) in jeder Schwenkstellung der Seitenteile (6, 7) zumindest teilweise abzudecken.

2. Eckelement nach Anspruch 1, **dadurch gekennzeichnet, dass** die mindestens eine Blende (12, 13, 14) mit dem mindestens einen Scharnier (8) verbunden ist.

3. Eckelement nach einem der vorgenannten Ansprüche, **gekennzeichnet durch** mindestens zwei Blenden (12, 13, 14), die relativ zueinander um die Schwenkachse (9) verschwenkbar sind.

4. Eckelement nach einem der vorgenannten Ansprüche, **dadurch gekennzeichnet, dass** die mindestens eine Blende (12, 13, 14) einen kreisbogenförmig um die Schwenkachse (9) ausgeführten Blendenflügel (16, 17) aufweist.

5. Eckelement nach Anspruch 4, **dadurch gekennzeichnet, dass** die Seitenteile (6, 7) an die Kreisbogenform des Blendenflügels (16, 17) der mindestens einen Blende (12, 13, 14) angepasste Blenden-Aufnahmen (40) aufweisen.

6. Eckelement nach einem der vorgenannten Ansprüche, **gekennzeichnet durch** drei Blenden (12, 13, 14), wobei zwei Seiten-Blenden (12, 13) der drei Blenden (12, 13, 14) jeweils einen entlang eines gemeinsamen Seiten-Kreisbogens um die Schwenkachse (9) ausgebildeten Seiten-Blendenflügel (16) aufweisen und eine Mitten-Blende (14) der drei Blenden (12, 13, 14) einen entlang eines Mitten-Kreisbogens um die Schwenkachse (9) ausgebildeten Mitten-Blendenflügel (17) aufweist, wobei ein Radius des Mitten-Kreisbogens sich von einem Radius des Seiten-Kreisbogens unterscheidet.

7. Eckelement nach einem der vorgenannten Ansprüche, **gekennzeichnet durch** mindestens eine Mitnehmervorrichtung (28, 29, 30, 31, 32, 33, 34, 35) je Blende (12, 13, 14), wobei jede Mitnehmervorrichtung zur Mitnahme der jeweiligen Blende (12, 13, 14) beim Verschwenken der Seitenteile ausgelegt ist, falls ein zwischen den Seitenteilen (6, 7) definierter Öffnungswinkel (b) kleiner als ein für die jeweilige Blende (12, 13, 14) vorgegebener Mitnahmewinkel ist.

8. Eckelement nach Anspruch 7, **dadurch gekennzeichnet, dass** jede Mitnehmervorrichtung (28, 29, 30, 31, 32, 33, 34, 35) durch Anschläge in dem mindestens einen Scharnier (8) ausgebildet ist.

9. Eckelement nach einem der vorgenannten Ansprüche, **dadurch gekennzeichnet, dass** die Seitenteile (6, 7) im Bereich des mindestens einen Scharniers (8) jeweils einen Befestigungsblock (19, 20) aufweisen.

10. Eckelement nach Anspruch 9, **dadurch gekennzeichnet, dass** der mindestens eine Befestigungsblock (19, 20) je Seitenteil (6, 7) in Richtung der Schwenkachse (21) übereinandergestapelte Blechzuschnitte (21, 23, 28, 35) aufweist.

11. Eckelement nach einem der vorgenannten Ansprüche, **dadurch gekennzeichnet, dass** die Seitenteile (6, 7) Befestigungselemente (26) zum Befestigen weiterer Komponenten der Strahlenschutzwand (1; 100) aufweisen.

12. Eckelement nach einem der vorgenannten Ansprüche, **dadurch gekennzeichnet, dass** die mindestens eine Blende (12, 13, 14) ein Strahlenschutzmaterial, insbesondere ein Laserschutzmaterial aufweist.

13. Strahlenschutzwand, insbesondere Laserschutzwand, umfassend mindestens zwei Wandelemente (2), die über ein schwenkbares Eckelement (3) nach einem der vorgenannten Ansprüche verbunden sind.

14. Strahlenschutzwand nach Anspruch 13 **gekennzeichnet durch** einen modularen Aufbau.
